# FASCICULE DE BREVET EUROPEEN

(11) **EP 3 297 709 B1**
(45) Date de publication et mention de la délivrance du brevet: **06.01.2021**
(21) Numéro de dépôt: 16733139.6
(22) Date de dépôt: 19.05.2016
(51) Int. Cl.: A61M 5/32, A61M 25/06, A61B 17/00, A61M 25/00

(54) **DISPOSITIF D'INJECTION DE PRODUIT INJECTABLE COMPORTANT UNE CANULE COULISSANT DANS UNE AIGUILLE ET SYSTÈME UTILISANT UN TEL DISPOSITIF**
INJEKTIONSVORRICHTUNG FÜR INJIZIERBARES MATERIAL MIT EINER GLEITKANÜLE IM INNEREN EINER NADEL UND SYSTEM MIT DERARTIGER VORRICHTUNG
INJECTABLE MATERIAL INJECTION DEVICE COMPRISING A CANNULA THAT SLIDES INSIDE A NEEDLE, AND SYSTEM USING SUCH A DEVICE

(30) Priorité: 19.05.2015 FR 1554466
(43) Date de publication de la demande: 28.03.2018
(73) Titulaire: Cumbo, Peter, 06140 Vence (FR)
(72) Inventeur: Cumbo, Peter, 06140 Vence (FR)
(74) Mandataire: Ipside
(86) Numéro de dépôt international: PCT/FR2016/051184
(87) Numéro de publication internationale: WO 2016/185145

(56) Documents cités:
- EP-A1- 1 736 192
- WO-A1-02/085444
- WO-A1-2017/031478
- WO-A2-2015/015127
- FR-A1- 2 308 346
- GB-A- 2 425 484
- US-A- 3 840 008
- US-A1- 2005 010 178
- US-A1- 2012 035 551
- US-A1- 2012 226 239

## Description

### DOMAINE TECHNIQUE DE L'INVENTION

La présente invention vise un dispositif d'injection de produit injectable et un système d'injection de produit injectable.

La présente invention peut être utilisée pour réaliser toute injection, à visée médicale ou esthétique, notamment pour les injections sous-cutanées. L'invention trouve une application particulièrement avantageuse dans l'injection de produit de comblement à visée esthétique.

### ÉTAT DE LA TECHNIQUE

Il est connu de l'art antérieur de réaliser des injections de produits de comblement, comme l'acide hyaluronique ou l'hydroxyapatite de calcium voire le collagène, sur le visage à l'aide d'une seringue simple comportant une aiguille creuse apte à perforer la peau. Le produit de comblement contenu dans la seringue est injecté sous la peau en divers endroits pour obtenir un effet esthétique souhaité. Cette méthode classique présente l'inconvénient de devoir réaliser de dix à quinze piqûres, afin de couvrir l'ensemble du visage et occasionne de ce fait une douleur et un inconfort important pour le patient. Les risques d'œdèmes et d'hématome sont de plus accrus dû au nombre important de piqûres.

Il est connu de l'art antérieur d'utiliser une canule pour réduire le nombre de piqûres nécessaire. Une perforation de la peau est d'abord effectuée au moyen d'une aiguille creuse de diamètre approprié, puis l'orifice cutané ainsi réalisé est repéré à l'apparition d'une goutte de sang. Une canule est ensuite insérée dans l'orifice cutané puis la canule est dirigée vers les différents sites d'injection du produit de comblement. Cette méthode présente l'inconvénient de souvent rendre l'introduction de la canule difficile, l'orifice créé à l'aide de l'aiguille étant régulièrement difficile à identifier par le praticien notamment s'il n'y a pas apparition d'une goutte de sang et la canule peut être difficile à insérer de sorte que le praticien soit obligé de s'y reprendre à plusieurs fois, causant un traumatisme tissulaire plus important et un risque accru de saignement, de douleur et d'œdème, sources d'inconfort pour le patient. Par exemple, WO2015/015127 divulgue un kit et une méthode pour injecter un produit dans le cadre d'une intervention esthétique, US 3,840,008 divulgue une seringue hypodermique pour injecter en toute sécurité un liquide dans les zones nerveuses et les vaisseaux encombrés d'un patient, et WO2017/031478 divulgue une aiguille et une canule intégrées pour la chirurgie plastique.

Aucun des systèmes actuels ne permet de répondre simultanément à tous les besoins requis, à savoir de permettre de pratiquer des injections de produits en des sites multiples au sein des tissus à partir d'un point de perforation cutané unique grâce à un geste simple : un seul dispositif et un seul geste.

### OBJET DE L'INVENTION

La présente invention vise à remédier à tout ou partie de ces inconvénients.

A cet effet, selon un premier aspect défini par la revendication 1 la présente invention vise un dispositif d'injection de produit injectable qui comporte une canule coulissant dans une aiguille hypodermique comportant une pointe biseautée, la canule comportant une embase configurée pour établir une liaison étanche.

On entend par produit injectable tout liquide et en particulier les liquides que l'on peut injecter dans le corps humain. Le produit peut être de l'acide hyaluronique, de l'hydroxyapatite de calcium, du collagène par exemple.

Grâce à ces dispositions, un opérateur peut, au travers d'un geste unique, perforer la peau d'un patient au moyen d'une aiguille creuse, puis déployer au sein des tissus une canule à extrémité mousse, atraumatique, afin d'y déposer un produit injectable. Ces dispositions suppriment l'étape consistant à tenter d'insérer la canule au travers de l'orifice pratiqué préalablement dans la peau au moyen d'une aiguille hypodermique indépendante, la canule étant ensuite déployée au travers de la lumière de l'aiguille guide. De plus, l'opérateur peut réaliser les opérations de piqûre de la peau par l'aiguille et d'insertion de la canule en conservant le dispositif au contact de la peau. Ces dispositions se distinguent des solutions de l'art antérieur dans lesquelles les opérations de piqûre et d'insertion de canule nécessitent de réaliser une piqûre à l'aide d'un premier appareil, puis de retirer le premier appareil et de déplacer ses mains pour se saisir d'un second appareil et réaliser l'étape d'insertion de la canule.

Selon la présente invention, la pointe de l'aiguille hypodermique comporte un biseau principal et un biseau arrière.

Grâce à ces dispositions, la perforation de la peau par le biseau principal de la pointe de l'aiguille est aisée, tout en étant atraumatique lorsque la canule est déployée, ceci grâce au biseau arrière permettant l'effacement de la pointe qui devient affleurante par rapport à la paroi de la canule. Ainsi, lors des manipulations de la canule, la pointe de l'aiguille n'est pas traumatisante pour les tissus.

Dans des modes de réalisation, l'aiguille hypodermique comporte un premier filetage et la canule comporte un deuxième filetage configuré pour être vissé au premier filetage.

Grâce à ces dispositions, l'aiguille est fixée à la canule. L'opérateur peut facilement accrocher et décrocher la canule à l'aiguille en vissant ou dévissant. Egalement, grâce à ces dispositions, l'opérateur peut contrôler, en vissant la canule vers l'intérieur de l'aiguille, la longueur de canule dépassant de la pointe de l'aiguille. Un contrôle précis de la longueur de canule dépassant de la pointe de l'aiguille est important notamment lors de l'étape d'insertion et de déplacement de la canule au sein des tissus.

Dans des modes de réalisation, le dispositif comporte un moyen de blocage configuré pour bloquer la canule par rapport à l'aiguille hypodermique sur une position prédéterminée.

Grâce à ces dispositions, le moyen de blocage permet d'empêcher le coulissement de l'aiguille par rapport à la canule. Le moyen de blocage permet de bloquer la canule par rapport à l'aiguille sur au moins une position prédéterminée.

Dans des modes de réalisation, le dispositif comporte au moins un repère visuel indiquant la longueur de la partie de la canule dépassant de la pointe de l'aiguille.

Grâce à ces dispositions, l'opérateur peut connaître la longueur de canule dépassant de la pointe de l'aiguille simplement en regardant le repère visuel. Le repère visuel est par exemple situé au niveau du filetage de la canule ou au niveau de l'extrémité d'insertion de la canule.

Dans des modes de réalisation, l'embase comporte un raccord pour seringue de type Luer Lock.

Grâce à ces dispositions, l'embase de la canule peut être connectée de manière étanche, notamment avec une seringue. Ces dispositions permettent un raccordement rapide, sûr et étanche entre le dispositif objet de l'invention et tout autre élément médical disposant d'un raccord de type Luer Lock. Un élément médical comportant un raccord de type Luer Lock peut être un tube à transfusion ou bien un tube à perfusion.

Dans des modes de réalisation, la canule comporte une extrémité d'insertion de forme arrondie.

Grâce à ces dispositions, l'extrémité qui est introduite dans la peau du patient présente une extrémité de forme ronde non biseautée et non-tranchante qui peut pénétrer les tissus sans léser les vaisseaux et filets nerveux notamment, réduisant l'inconfort du patient pendant la séance d'injection et diminuant le risque de survenue d'ecchymoses.

Selon un second aspect, la présente invention vise un système d'injection de produit qui comporte un dispositif d'injection de produit objet de la présente invention.

Les buts, avantages et caractéristiques particulières du système d'injection de produit portant le dispositif objet de la présente invention étant similaires à ceux du dispositif objet de la présente invention, ils ne sont pas rappelés ici.

### BRÈVE DESCRIPTION DES FIGURES

D'autres avantages, buts et caractéristiques particuliers de la présente invention ressortiront de la description non limitative qui suit d'au moins un mode de réalisation particulier des dispositifs, procédés et systèmes objets de la présente invention, en regard des dessins annexés, dans lesquels :
- la figure 1 représente, schématiquement et en vue de coté, un mode de réalisation particulier du dispositif objet de la présente invention,
- la figure 2 représente, schématiquement et en vue de coté, un mode de réalisation particulier du dispositif objet de la présente invention,
- la figure 3 représente, schématiquement et en coupe, un mode de réalisation particulier de la pointe de l'aiguille du dispositif objet de la présente invention,
- la figure 4 représente, schématiquement et en perspective, un mode de réalisation particulier de la pointe de l'aiguille du dispositif objet de la présente invention, et
- la figure 5 représente, schématiquement et en vue de coté, un mode de réalisation particulier d'un système portant le dispositif objet de la présente invention.

### DESCRIPTION DÉTAILLÉE DE MODES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note que le terme « un » est partout utilisé au sens de « au moins un ».

On note, dès à présent, que les figures ne sont pas à l'échelle.

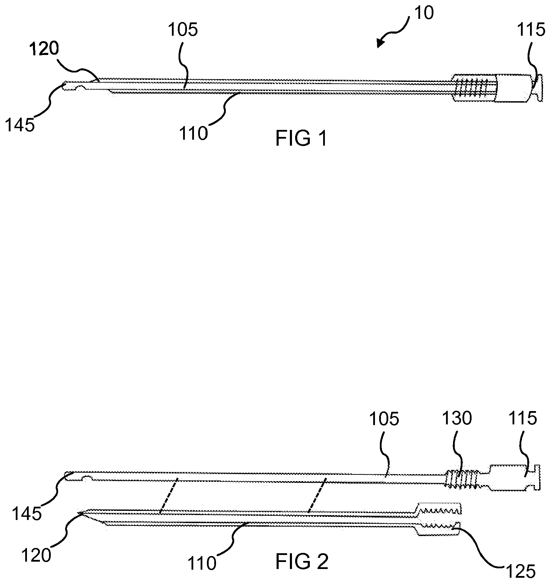

On observe, en figure 1, un dispositif 10 d'injection de produit injectable qui comporte une canule 105 coulissant dans une aiguille hypodermique 110 comportant une pointe 120 biseautée, la canule 105 comportant une embase 115 configurée pour établir une liaison étanche. La canule comporte un tube en matière plastique ou en métal, droit ou recourbé, permettant le passage de l'air ou d'un produit injectable à travers un orifice, naturel ou confectionné par le praticien. Préférentiellement, la canule 105 est métallique, de forme droite et allongée et configurée pour injecter un produit injectable. Dans des modes de réalisation, la canule 105 est formée d'acier, d'acier inoxydable ou de titane.

Dans des modes de réalisation, la canule 105 comporte une extrémité d'insertion 145 configurée pour être insérée au travers d'un trou pratiqué dans la peau et à l'autre extrémité une embase comportant un filetage 130. L'extrémité d'insertion 145 et le filetage 130 sont décrits en figure 2. Le tube de la canule a un diamètre prédéterminé configuré pour être adapté au produit à injecter. Dans des modes de réalisation, la canule 105 a un diamètre de 1,24 mm (abrévié de millimètre), 0,81 mm, 0,71 mm, 0,63 mm, 0,56 mm, 0,46 mm ou 0,30 mm. Dans des modes de réalisation préférentiels, la canule 105 a un diamètre compris entre 0,56 mm et 0,30 mm.

La canule 105 comporte une embase 115 configurée pour réaliser une liaison étanche avec une seringue, un matériel de transfusion ou de perfusion par exemple. Dans des modes de réalisation, l'embase 115 comporte un raccord pour seringue de type Luer. On rappelle ici que les raccords Luer sont des connecteurs étanches standardisés notamment décrits dans les normes européennes NF EN 20594-1 publiée en 1993 et française ISO 594-1 publiée en 1986. Dans d'autres modes de réalisation, l'embase 115 comporte un raccord de type Luer inversé.

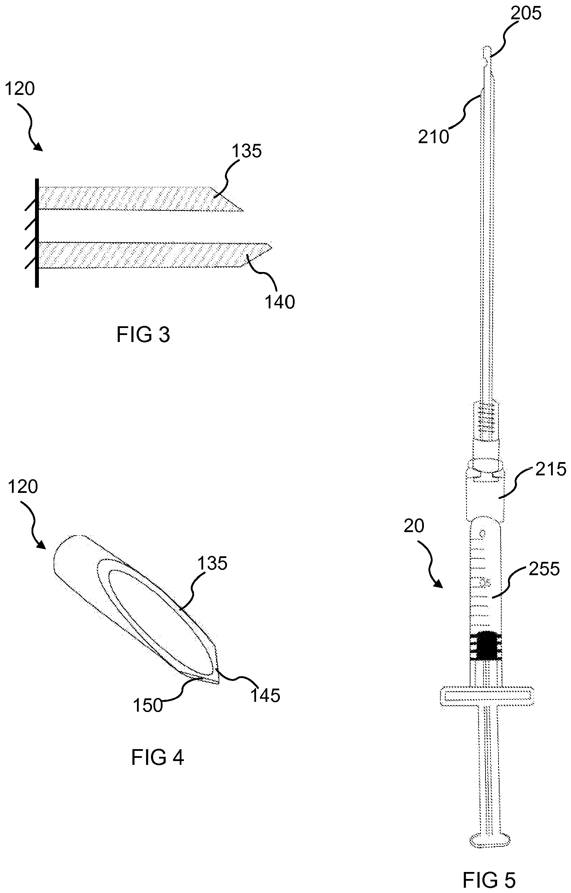

L'aiguille hypodermique 110 comporte une pointe 120, représentée en figures 3 et 4, configurée pour traverser la peau par perforation. L'aiguille hypodermique 110 comporte un tube en matière plastique ou en métal. Préférentiellement, l'aiguille hypodermique 110 est un tube métallique de diamètre supérieur à celui de la canule. Dans des modes de réalisation, l'aiguille hypodermique 110 comporte un filetage 125 décrit en figure 2.

Les deux tubes creux de la canule 105 et de l'aiguille hypodermique 110 sont arrangés en cercles concentriques, la canule 105 étant contenue dans l'aiguille hypodermique 110. La canule 105 est apte à coulisser en translation par rapport à la seringue hypodermique 110 dans le sens de la liaison glissière formée par l'ensemble canule 105 et aiguille hypodermique 110.

Préférentiellement, la canule 105 est plus longue que l'aiguille hypodermique 110. Dans des modes de réalisation, la longueur de la canule 105 est comprise entre 25 mm et 75 mm. Préférentiellement la longueur de l'aiguille hypodermique 110 est de 5mm inférieur à la longueur de la canule 105.

Dans des modes de réalisation, l'aiguille hypodermique 110 est très courte. Dans ce mode de réalisation, la longueur de l'aiguille hypodermique 110 est comprise entre cinq et dix millimètres, et permet de laisser dépasser une longueur importante de canule 105 de la pointe 120 de l'aiguille hypodermique 110. Dans des modes de réalisation, la longueur de canule 105 pouvant dépasser de la pointe 120 de l'aiguille hypodermique est supérieure à 25 mm.

Dans des modes de réalisation, le dispositif 10 comporte au moins un repère visuel indiquant la longueur de la partie de la canule dépassant de la pointe 120 de l'aiguille hypodermique 110.

On observe en figure 2, une vue éclatée du dispositif 10 d'injection de produit qui comporte une canule 105 coulissante et une aiguille hypodermique 110.

La canule 105 comporte une extrémité d'insertion 145 configurée pour être glissée sous la peau et pour injecter un produit injectable. Dans des modes de réalisation, l'extrémité d'insertion 145 comporte une ouverture configurée pour laisser sortir le produit injecté par la canule. Dans des modes de réalisation, l'ouverture de l'extrémité d'insertion 145 se trouve en retrait du bout de l'extrémité d'insertion 145. Dans des modes de réalisation, la distance entre le bout de l'extrémité d'insertion 145 et l'ouverture est supérieure à un millimètre.

Dans des modes de réalisation préférentiels, la canule 105 comporte une extrémité d'insertion 145 de forme arrondie.

Préférentiellement l'extrémité d'insertion 145 de la canule 105 est configurée pour être atraumatique. L'extrémité d'insertion 145 de la canule 105 est un arrondi qui ne présente pas de bord tranchant.

Dans des modes de réalisation, l'aiguille hypodermique 110 comporte un premier filetage 125 et la canule comporte un deuxième filetage 130 configuré pour être vissé au premier filetage 125. Le premier filetage 125 et le deuxième filetage 130 sont configurés pour pouvoir visser la canule 105 d'une position de perforation dans laquelle la canule 105 ne dépasse pas de la point de l'aiguille hypodermique 110 à une position d'injection dans laquelle l'extrémité d'insertion 145 de la canule 105 dépasse de la pointe de l'aiguille hypodermique 110.

Dans des modes de réalisation préférentiels, la canule 105 comporte un filetage 130 sur sa surface externe et l'aiguille 110 comporte un filetage 125 sur sa surface interne. En d'autres termes, le filetage 125 sur la surface interne de l'aiguille hypodermique 110 est un taraudage.

Dans des modes de réalisation, l'aiguille 110 comporte un filetage sur sa surface externe et la canule 105 comporte un filetage sur sa surface interne.

Dans des modes de réalisation, le premier filetage 125 et le deuxième filetage 130 sont des filetages métriques triangulaires. Dans d'autres modes de réalisation le premier filetage 125 et le deuxième filetage 130 sont des filetages hélicoïdaux.

Préférentiellement le premier filetage 125 et le deuxième filetage 130 ont une longueur comprise entre 3 mm et 15 mm.

Dans des modes de réalisation, le dispositif 10 comporte un moyen de blocage configuré pour bloquer la canule 105 par rapport à l'aiguille hypodermique 110 sur au moins une position prédéterminée. Dans des modes de réalisation, le moyen de blocage est un épaulement sur l'aiguille hypodermique 110. Dans des modes réalisations préférentiels, les positions prédéterminées de blocage de la canule 105 dans l'aiguille hypodermique 110 par le moyen de blocage incluent au moins une première position de blocage dans laquelle la canule ne dépasse pas de la pointe de l'aiguille hypodermique 110 et au moins une position de blocage dans laquelle la longueur de la partie de la canule 105 dépassant de la pointe de l'aiguille hypodermique 110 est supérieur à 2 mm.

Le moyen de blocage est configuré pour empêcher le mouvement de translation entre la canule 105 et la seringue hypodermique 110. Dans des modes de réalisation, le moyen de blocage est une pince articulée maintenue sous tension par un ressort. Ainsi, l'opérateur peut presser les extrémités de la pince pour permettre le mouvement en translation de la canule 105 par rapport à la seringue 110 ou relâcher pour empêcher le mouvement en translation de la canule 105 par rapport à la seringue 110.

On observe en figure 3, une vue en coupe d'un mode de réalisation de la pointe 120 biseautée de l'aiguille 110 du dispositif 10. La pointe 120 de l'aiguille hypodermique comporte un biseau principal 135 et un biseau arrière 140. Dans des modes de réalisation, on utilise un outil de meulage comportant des moyens abrasifs pour usiner le biseau principal, le biseau principal 135 étant taillé sur la surface de l'aiguille hypodermique 110 selon un angle prédéterminé par rapport à l'axe de l'aiguille hypodermique 110.

Préférentiellement, le biseau principal 135 a une surface plus grande que le biseau arrière. Préférentiellement, le biseau principal 135 a une surface plane. Dans des modes de réalisation, l'angle défini par le biseau principal 135 par rapport à l'axe de l'aiguille hypodermique 110 est inférieur à quarante degrés. Préférentiellement, le biseau arrière 140 est taillé sur une partie de l'aiguille hypodermique 110 diamétralement opposée à celle du biseau principal 135.

On observe en figure 4, une vue en perspective d'un mode de réalisation de la pointe 120 biseautée de l'aiguille 110 du dispositif 10 objet de la présente invention. Dans des modes de réalisation, la pointe de l'aiguille hypodermique 110 comporte un biseau principal 135 et deux biseaux secondaires, 145 et 150, taillés de par et d'autre de la partie fine formée par le biseau principal. En d'autres termes, les biseaux secondaires 145 et 150 sont des facettes disposées de part et d'autre du biseau principal.

On observe en figure 5, un mode de réalisation du système 20 d'injection de produit qui comporte le dispositif d'injection de produit objet de la présente invention. Préférentiellement, le système 20 d'injection de produit comporte une canule 205 coulissant dans une aiguille hypodermique 210 comportant une pointe biseautée, la canule 205 comportant une embase 215 configurée pour établir une liaison étanche et une seringue 255.

Dans des modes de réalisation (non représentés), le système 20 d'injection de produit injectable comporte une canule 205 coulissant dans une aiguille hypodermique 210 comportant une pointe biseautée, la canule 205 comportant une embase 215 configurée pour établir une liaison étanche et un tube souple de perfusion ou de transfusion.

Dans des modes de réalisation (non représentés), le système 20 comporte un moyen de poussée de la canule permettant de faire coulisser la canule 205 en translation par rapport à la seringue hypodermique 210 dans le sens de la liaison glissière formée par l'ensemble canule 205 et aiguille hypodermique 210.

Dans des modes de réalisation (non représentés), le système 20 comporte une indication visuelle représentative de l'orientation du plan défini par le biseau principal. Préférentiellement, l'indication visuelle est présente sur l'embase 215.

## Revendications

1. Dispositif (10) d'injection de produit injectable comprenant une canule (105) coulissant dans une aiguille hypodermique (110) présentant une pointe (120) biseautée, la canule (105) comportant une embase (115) configurée pour établir une liaison étanche, ledit dispositif d'injection comprenant en outre un moyen de blocage configuré pour bloquer la canule (105) par rapport à l'aiguille hypodermique (110) sur au moins une position prédéterminée pour empêcher le mouvement de translation entre la canule (105) et l'aiguille hypodermique (110), **caractérisé en ce que** l'aiguille hypodermique comporte un biseau principal (135) et un biseau arrière (140).

2. Dispositif (10) d'injection selon la revendication 1, **caractérisé en ce que** le biseau arrière (140) est taillé sur une partie de l'aiguille hypodermique (110) diamétralement opposée à celle du biseau principal (135).

3. Dispositif (10) d'injection selon la revendication 1 ou la revendication 2, **caractérisé en ce que** la pointe de l'aiguille hypodermique (110) comporte un biseau principal (135) et deux biseaux secondaires (145, 150) taillés de part et d'autre de la partie fine formée par le biseau principal (135).

4. Dispositif (10) d'injection selon l'une quelconque des revendications précédentes, dans lequel l'aiguille hypodermique (110) comporte un premier filetage (125) et la canule comporte un deuxième filetage (130) configuré pour être vissé au premier filetage (125).

5. Dispositif (10) d'injection selon l'une quelconque des revendications précédentes, dans lequel le moyen de blocage est un épaulement sur l'aiguille hypodermique (110).

6. Dispositif (10) d'injection selon l'une quelconque des revendications 1 ou 5, dans lequel les positions prédéterminées de blocage de la canule (105) dans l'aiguille hypodermique (110) par le moyen de blocage incluent au moins une première position de blocage dans laquelle la canule ne dépasse pas de la pointe de l'aiguille hypodermique (110) et au moins une position de blocage dans laquelle la longueur de la partie de la canule (105) dépassant de la pointe de l'aiguille hypodermique (110) est supérieure à deux millimètres.

7. Dispositif (10) d'injection selon l'une quelconque des revendications 1 à 6, qui comporte au moins un repère visuel indiquant la longueur de la partie de la canule dépassant de la pointe (120) de l'aiguille hypodermique (110).

8. Dispositif (10) d'injection selon l'une quelconque des revendications 1 à 7, dans lequel l'embase (115) comporte un raccord pour seringue de type Luer Lock.

9. Dispositif (10) d'injection selon l'une quelconque des revendications 1 à 8, dans lequel la canule (105) comporte une extrémité d'insertion (145) de forme arrondie.

10. Système (20) d'injection de produit injectable qui comporte un dispositif d'injection de produit injectable selon l'une quelconque des revendications 1 à 9.

## Patentansprüche

1. Injektionsvorrichtung (10) für ein injizierbares Produkt, umfassend eine Kanüle (105), die in einer hypodermischen Nadel (110) gleitet, die eine abgeschrägte Spitze (120) aufweist, wobei die Kanüle (105) einen Sockel (115) umfasst, der ausgestaltet ist, eine dichte Verbindung herzustellen, wobei die Injektionsvorrichtung des Weiteren ein Sperrmittel umfasst, das ausgestaltet ist, die Kanüle (105) in Bezug auf die hypodermische Nadel (110) auf mindestens einer vorbestimmten Position zu sperren, um die Verschiebungsbewegung zwischen der Kanüle (105) und der hypodermischen Nadel (110) zu verhindern, **dadurch gekennzeichnet, dass** die hypodermische Nadel eine Hauptabschrägung (135) und eine hintere Abschrägung (140) umfasst.

2. Injektionsvorrichtung (10) nach Anspruch 1, **dadurch gekennzeichnet, dass** die hintere Abschrägung (140) auf einen Teil der hypodermischen Nadel (110) zugeschnitten ist, der jenem der Hauptabschrägung (135) diametral entgegengesetzt ist.

3. Injektionsvorrichtung (10) nach Anspruch 1 oder Anspruch 2, **dadurch gekennzeichnet, dass** die Spitze der hypodermischen Nadel (110) eine Hauptabschrägung (135) und zwei Nebenabschrägungen (145, 150) umfasst, die auf jeder Seite des feinen Teils zugeschnitten sind, der von der Hauptabschrägung (135) gebildet wird.

4. Injektionsvorrichtung (10) nach einem der vorstehenden Ansprüche, wobei die hypodermische Nadel (110) ein erstes Gewinde (125) umfasst und die Kanüle ein zweites Gewinde (130) umfasst, das ausgestaltet ist, auf das erste Gewinde (125) geschraubt zu werden.

5. Injektionsvorrichtung (10) nach einem der vorstehenden Ansprüche, wobei das Sperrmittel ein Vorsprung auf der hypodermischen Nadel (110) ist.

6. Injektionsvorrichtung (10) nach einem der Ansprüche 1 oder 5, wobei die vorbestimmten Sperrpositionen der Kanüle (105) in der hypodermischen Nadel (110) mithilfe des Sperrmittels mindestens eine erste Sperrposition einschließen, in der die Kanüle nicht über die Spitze der hypodermischen Nadel (110) hinausragt, und mindestens eine Sperrposition, in der die Länge des Teils der Kanüle (105), der über die Spitze der hypodermischen Nadel (110) hinausragt, größer ist als zwei Millimeter.

7. Injektionsvorrichtung (10) nach einem der Ansprüche 1 bis 6, die mindestens eine visuelle Markierung umfasst, welche die Länge des Teils der Kanüle, der über die Spitze (120) der hypodermischen Nadel (110) hinausragt, anzeigt.

8. Injektionsvorrichtung (10) nach einem der Ansprüche 1 bis 7, wobei der Sockel (115) einen Anschluss für Spritzen vom Typ Luer Lock umfasst.

9. Injektionsvorrichtung (10) nach einem der Ansprüche 1 bis 8, wobei die Kanüle (105) eine Einführextremität (145) mit abgerundeter Form umfasst.

10. Injektionssystem (20) für ein injizierbares Produkt, das eine Injektionsvorrichtung für ein injizierbares Produkt nach einem der Ansprüche 1 bis 9 umfasst.

## Claims

1. Device (10) for injecting injectable product, comprising a cannula (105) sliding in a hypodermic needle (110) having a bevelled tip (120), the cannula (105) including a base (115) configured to establish a sealed connection, said injection device further comprising a locking means configured to lock the cannula (105) with respect to the hypodermic needle (110) on at least one predetermined position in order to prevent translation movement between the cannula (105) and the hypodermic needle (110), **characterised in that** the hypodermic needle includes a main bevel (135) and a rear bevel (140).

2. Injection device (10) according to claim 1, **characterised in that** the rear bevel (140) is cut on a part of the hypodermic needle (110) diametrically opposite to that of the main bevel (135).

3. Injection device (10) according to claim 1 or claim 2, **characterised in that** the tip of the hypodermic needle (110) includes a main bevel (135) and two secondary bevels (145, 150) cut on either side of the fine part formed by the main bevel (135).

4. Injection device (10) according to any one of the preceding claims, wherein the hypodermic needle (110) includes a first thread (125) and the cannula includes a second thread (130) configured to be screwed to the first thread (125).

5. Injection device (10) according to any one of the preceding claims, wherein the locking means is a shoulder on the hypodermic needle (110).

6. Injection device (10) according to either one of claims 1 or 5, wherein the predetermined positions of locking of the cannula (105) in the hypodermic needle (110) by the locking means include at least one first locking position wherein the cannula does not project beyond the tip of the hypodermic needle (110) and at least one locking position wherein the length of the part of the cannula (105) projecting beyond the tip of the hypodermic needle (110) is greater than two millimetres.

7. Injection device (10) according to any one of claims 1 to 6, which includes at least one visual mark indicating the length of the part of the cannula projecting beyond the tip (120) of the hypodermic needle (110).

8. Injection device (10) according to any one of claims 1 to 7, wherein the base (115) includes a coupling for a syringe of the Luer Lock type.

9. Injection device (10) according to any one of claims 1 to 8, wherein the cannula (105) includes a rounded-shaped insertion end (145).

10. System (20) for injecting injectable product that includes an injectable-product injection device according to any one of claims 1 to 9.
